# EUROPÄISCHE PATENTSCHRIFT

(11) **EP 3 271 465 B1**
(45) Veröffentlichungstag und Bekanntmachung des Hinweises auf die Patenterteilung: **11.09.2019**
(21) Anmeldenummer: 16718991.9
(22) Anmeldetag: 03.03.2016
(51) Int. Cl.: C12P 7/22, C07C 39/20, C12P 13/22, C12P 7/42

(54) **VERFAHREN ZUR HERSTELLUNG VON P-VINYLPHENOLEN**
METHOD FOR PRODUCING P-VINYLPHENOLS
PROCÉDÉ DE PRODUCTION DE P-VINYLPHÉNOLS

(30) Priorität: 10.03.2015 AT 1302015
(43) Veröffentlichungstag der Anmeldung: 24.01.2018
(73) Patentinhaber: Universität Graz, 8010 Graz (AT)
(72) Erfinder: KROUTIL, Wolfgang, 8042 Graz (AT); BUSTO, Eduardo, 33012 Oviedo (ES); SIMON, Robert, 82362 Weilheim OBB (DE)
(74) Vertreter: Ellmeyer, Wolfgang
(86) Internationale Anmeldenummer: PCT/AT2016/050051
(87) Internationale Veröffentlichungsnummer: WO 2016/141397

(56) Entgegenhaltungen:
- EP-A1- 2 641 964
- WO-A2-03/099233
- WO-A2-2004/092344
- US-A1- 2003 079 255
- US-A1- 2004 248 267
- EDUARDO BUSTO ET AL: "Vinylation of Unprotected Phenols Using a Biocatalytic System", ANGEWANDTE CHEMIE, Bd. 127, Nr. 37, 29. Juli 2015 (2015-07-29), Seiten 11049-11052, XP055283233, DE ISSN: 0044-8249, DOI: 10.1002/ange.201505696

## Beschreibung

Die Erfindung betrifft ein biokatalytisches Verfahren zur Herstellung von *p*-Vinylphenolen.

### STAND DER TECHNIK

Vinylphenol-Derivate dienen als nützliche Bausteine in der Polymerchemie und können beispielsweise zum Aufbau dielektrischer Schichten bei der Herstellung chemischer und biologischer Sensoren eingesetzt werden. Halogenierte Derivate finden beispielsweise Anwendung bei der Herstellung von Flammschutzmitteln sowie von Chalkonen, eine bekannte Klasse organischer Verbindungen mit einem breiten Spektrum an biologischen Aktivitäten.

Eine selektive para-Vinylierung nichtaktivierter Phenole zu *para-* oder 4-Vinylphenolen ist nicht bekannt. So führt eine direkte Vinylierung nichtaktivierter Arene unter Verwendung von Zinnkatalysatoren zu selektiver ortho-Derivatisierung (Yamaguchi et al., J. Am. Chem. Soc. 117, 1151-1152 (1995)), während die Katalyse mit LewisSäuren, wie z.B. GaCl₃, ein Gemisch der ortho- und para-Regioisomere liefert (Yamaguchi et al., Angew. Chem. Int. Ed. 36, 1313-1315 (1997)). Beide Strategien wurden jedoch nicht an Phenolderivaten durchgeführt. Vielmehr wurden *p*-Vinylphenole mittels Stille-Kupplung unter Verwendung von entsprechenden *p*-Bromphenolen und äußerst toxischen Vinylzinn-Reagenzien synthetisiert (Littke et al., J. Am. Chem. Soc. 124, 6343-6348 (2002)).

Alternativ dazu können *p*-Vinylphenole aus den entsprechenden 4-Hydroxybenzaldehyden mittels Heck-Reaktion unter Phosphoniumsalz-Katalyse (Chen et al., Tetrahedron 69, 653-657 (2013)) oder durch Knoevenagel-Kondensation in der Variante nach Doebner mittels Katalyse mit sekundären Aminen unter Mikrowellenstrahlung (Sinha et al., Tetrahedron 63, 960-965 (2007)) hergestellt werden.

Biokatalytische Ansätze sind hingegen auf die Decarboxylierung von *p*-Cumarsäuren (4-Hydroxyzimtsäuren) unter Verwendung von ionischen Flüssigkeiten (Sharma et al., Adv. Synth. Catal. 350, 2910-2920 (2008)), Phenolsäure-Decarboxylasen (PAD) (Wuensch et al., Org. Lett. 14, 1974-1977 (2012)) oder spezieller p-Hydroxyzimtsäure- bzw. *p*-Cumarsäure-Decarboxylasen (pHCA-DC bzw. pCA-DC oder kurz PDC), beispielsweise einer PDC von *L. plantarum* (Rodriguez et al., Proteins 78, 1662-1676 (2010) und Jung et al., Appl. Microbiol. Biotechnol. 97,1501 (2013)), mitunter in einem zweiphasigen System (Ben-Bassat et al., Org. Process Res. Dev. 11, 278-285 (2007)) beschränkt:

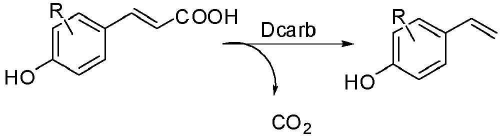

Dazu muss in der Regel jedoch die entsprechende Zimtsäure aus dem jeweiligem Aldehyd synthetisiert werden, was kostspielig ist. Auch die bakterielle Produktion von *p*-Vinylphenolen aus Glucose unter Verwendung genetisch manipulierter Bakterien, die eine fungale Phenyl-Ammoniak-Lyase (PAL) und eine bakterielle *p*-Hydroxyzimtsäure-Decarboxylase (PDC) co-exprimieren, durch Eliminierung von Ammoniak aus Tyrosin wurde berichtet. Dabei sind jedoch die Produktivitäten gering (0,4 g/l), die Reaktion ist auf dieses Substrat beschränkt, und die Mengen an Nebenprodukten wie Phenylalanin (0,5 g/l) und Zimtsäure sind erheblich (Qi et al., Metabolic Engin. 9, 268-276 (2007)).

Weiters ist die biokatalysierte Synthese von Tyrosinderivaten, ausgehend von Phenolen bekannt:

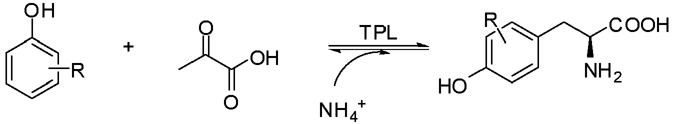

In Kroutil et al., Adv. Synth. Catal. 352, 731-736 (2010), einer Publikation der Arbeitsgruppe der Erfinder, wird diese Reaktion für eine Reihe von Phenolderivaten, darunter die Substituenten F, Cl, Br, CH₃ und OCH₃ an Position 2 oder 3 des Phenols, und unter Verwendung einer Tyrosin-Phenol-Lyase (TPL) von *Citrobacter freundii* offenbart.

Ebenfalls bekannt ist die enzymatisch katalysierte Eliminierung von Ammoniak aus Tyrosin mittels Tyrosin-Ammoniak-Lyasen (TAL) oder Phenyl-Ammoniak-Lyasen (PAL) zum Erhalt von Zimtsäure:

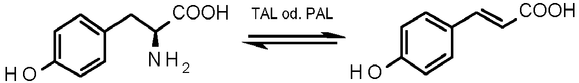

Siehe Kyndt et al., FEBS Letters 512, 240 (2002), sowie Louie et al., Chem. Biol. 13(12), 1327-1338 (2006). Für Derivate von Tyrosin wurde diese Reaktion jedoch bisher nicht berichtet.

Vor diesem Hintergrund war das Ziel der Erfindung die Entwicklung eines verbesserten biokatalysierten Verfahrens zur Herstellung *p*-Vinylphenolen, das eine rasche und kostengünstige Herstellung derselben ermöglicht.

### OFFENBARUNG DER ERFINDUNG

Dieses Ziel erreicht die Erfindung durch Bereitstellung eines biokatalytischen Verfahrens zur Herstellung von *p*-Vinylphenolen, umfassend eine dreistufige Eintopfreaktion gemäß nachstehendem Reaktionsschema: 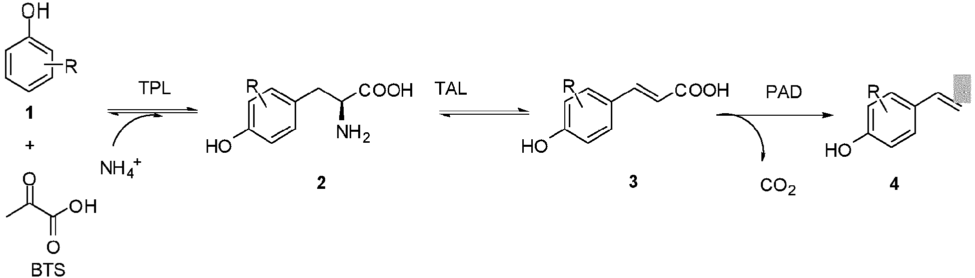 wobei
a) ein gegebenenfalls substituiertes Phenol **1** mittels katalytischer Wirkung einer Tyrosin-Phenol-Lyase (TPL) und in Gegenwart von Ammoniumionen mit Brenztraubensäure (BTS) zum gegebenenfalls substituierten Tyrosin **2** verbunden wird,
b) aus dem Tyrosin **2** mittels katalytischer Wirkung einer Tyrosin-Ammoniak-Lyase (TAL) Ammoniak eliminiert wird, um eine gegebenenfalls substituierte *p*-Cumarsäure **3** zu ergeben, und
c) die *p*-Cumarsäure **3** mittels katalytischer Wirkung einer Phenolsäure-Decarboxylase (PAD) einer Decarboxylierung unterzogen wird, um das gewünschte, gegebenenfalls substituierte *p*-Vinylphenol **4** zu ergeben;
d) wobei das entstehende CO₂ aus dem Reaktionssystem entfernt wird, um das chemische Gleichgewicht aller drei Reaktionsschritte in Richtung der Produkte zu verschieben.

Die Erfindung beruht dabei auf der Verknüpfung dreier an sich bekannter Einzelreaktionsschritte und den überraschenden Erkenntnissen, dass nicht nur alle drei in einer Eintopfreaktion im Wesentlichen gleichzeitig durchgeführt werden können, ohne einander in nennenswertem Ausmaß zu inhibieren, sondern dass dabei durchwegs Umsätze von über 90 % erzielbar sind - und das auch mit substituierten Phenolen als Ausgangssubstrat. Dies war vor allem aufgrund der Tatsache unerwartet, dass sowohl das Edukt als auch alle Produkte formal Phenole sind und somit die Wahrscheinlichkeit einer wechselseitigen inhibierenden Wirkung in den drei Einzelreaktionen hoch war.

Mittels Durchführung der drei Reaktionsschritte als Eintopfreaktion und Verschiebung des chemischen Gleichgewichts der Gesamtreaktion aufgrund der Eliminierung des bei der Decarboxylierung in Schritt c) frei werdenden Kohlendioxids aus dem System gelang es den Erfindern nach Optimierung der Reaktionsbedingungen sogar, Gesamtumsätze (über alle drei Stufen gerechnet) von durchwegs über 97 % zu erzielen, wie dies die späteren Ausführungsbeispiele belegen, womit keineswegs zu rechnen war.

Durch die vorliegende Erfindung wird somit ein Verfahren bereitgestellt, das eine selektive, äußerst rasche und nahezu vollständige Umsetzung von Phenolen zu *p*-Vinylphenolen in hoher Reinheit ermöglicht, was auf herkömmlichem organisch-chemischem Wege bisher nicht möglich war.

Die erfindungsgemäße Eintopfreaktion wird in bevorzugten Ausführungsformen bei einem pH von etwa 8 bis 9, noch bevorzugter bei etwa pH 8, durchgeführt, wie die Erfinder durch Variation des pH-Werts festgestellt haben. Auch in unter diesem Aspekt sind die ausgezeichneten Gesamtumsätze höchst überraschend, da das pH-Optimum der beiden ersten Reaktionsschritte jeweils über 10 liegt, während die Decarboxylierung bei saurem bis neutralem pH deutlich rascher und vollständiger vonstatten geht als im alkalischen Milieu und für die Einzelreaktion ab pH 9 kaum noch Umsatz zu beobachten war, wie erneut die späteren Beispiele belegen. Dieses Ergebnis für die Einzelreaktion deckt sich mit der eingangs zitierten Fachliteratur (Rodriguez et al., s.o.; Jung et al., s.o.), wo die biokatalysierte Decarboxylierung mittels PAD beispielsweise bei pH 6,5 bzw. 7,0 durchgeführt wird, obwohl Jung et al. (s.o.) ein pH-Optimum der von ihnen eingesetzte PAD bei pH 5,8 offenbaren.

Die Durchführung der erfindungsgemäßen Eintopfreaktion bei pH 8 hat sich aber auch aus dem Grund als optimal erwiesen, da bei diesem pH-Wert ein nichttoxischer KPi-Puffer (Kaliumphosphat) als wässriges Lösungsmittel eingesetzt werden kann, während bei pH 9 toxischere Reagenzien wie etwa Sulfonsäuresalze (z.B. CHES-Puffer: N-Cyclohexyl-2-aminoethansulfonsäure) verwendet werden müssten.

In weiteren bevorzugten Ausführungsformen wird in Schritt b) eine Tyrosin-Ammoniak-Lyase (TAL) als Katalysator eingesetzt, da die Erfinder mit den untersuchten Phenyl-Ammoniak-Lyasen (PAL) kaum Umsätze beobachten konnten. Die Tyrosin-Ammoniak-Lyase (TAL) wird vorzugsweise in Form von Ganzzellen, die das rekombinante Enzym enthalten, eingesetzt, da diese ausreichend hohe Aktivität im erfindungsgemäßen Verfahren zeigen und aufwändiges und mitunter verlustreiches Isolieren des Enzyms aus einer Zellkultur daher vermieden werden kann.

Dassselbe gilt für die gemäß vorliegender Erfindung vorzugsweise in Schritt c) als Katalysator eingesetzte Ferulasäure-Decarboxylase (FAD), die somit besonders bevorzugt ebenfalls in Form von Ganzzellen, die das rekombinante Enzym enthalten, eingesetzt wird.

In weiteren bevorzugten Ausführungsformen wird neben einem wässrigen Puffersystem mit entsprechendem pH ein mit Wasser nicht mischbares Co-Lösungsmittel eingesetzt, vorzugsweise Diethylether, besonders bevorzugt 5 % Diethylether, bezogen auf das wässrige Puffersystem, um den Umsatz der Gesamtreaktion weiter zu erhöhen.

Das Substitutionsmuster am Ausgangsphenol **1** ist nicht speziell eingeschränkt, solange die para-Position unbesetzt ist und der oder die Substituenten die enzymatischen Reaktionen der Schritte a) bis c) nicht nachteilig beeinflussen. Wie sich in den späteren Beispielen gezeigt hat, funktioniert die vorliegende Erfindung sowohl mit Substituenten, die die Elektronendichte im aromatischen Ring verringern, wie z.B. F, Cl und Br, als auch mit solchen, die sie erhöhen, wie z.B. Alkyl oder Alkoxy. Die Größe der Substituenten sollte dabei ein bestimmtes Maß nicht überschreiten, um die Koordination mit dem jeweiligen Enzym, insbesondere in Schritt a), nicht zu behindern, weswegen Kohlenwasserstoffreste mit nicht mehr als 20 Kohlenstoffatomen zu bevorzugen sind, noch bevorzugter Kohlenwasserstoffreste mit nicht mehr als 10 oder nicht mehr als 6 Kohlenstoffatomen. Besonders bevorzugt sind der oder die Substituenten an der ortho- oder meta-Position des Phenols **1** aus Halogenen sowie C₁₋₆-Alkyl- und C₁₋₆-Alkoxy-Resten ausgewählt, wie z.B. F, Cl, Br oder (O)CH₃.

### KURZBESCHREIBUNG DER ZEICHNUNGEN

Nachstehend wird die Erfindung unter Bezugnahme auf die beiliegenden Zeichnungen näher beschrieben, die im Einzelnen Folgendes darstellen:
Fig. 1 den Umsatz in Schritt a) bei unterschiedlichem pH;
Fig. 2 den Umsatz in Schritt b) bei unterschiedlichem pH;
Fig. 3 den Umsatz in Schritt c) bei unterschiedlichem pH;
Fig. 4 den zeitlichen Verlauf der Gesamtreaktion bei unterschiedlichem pH;
Fig. 5 die Mengen an Edukt und Produkten bei pH 7;
Fig. 6 die Mengen an Edukt und Produkten bei pH 10;
Fig. 7 den Umsatz der Gesamtreaktion mit unterschiedlichen Co-Lösungsmitteln; und
Fig. 8 die Mengen an Edukt und Produkten bei optimaler Reaktionsführung.

### BEISPIELE

Es folgen Ausführungsbeispiele der Erfindung, die zu Illustrationszwecken angegeben und nicht als Einschränkung des Schutzumfangs zu verstehen sind.

### Allgemeine Verfahren

Chemische Reagenzien wurden von verschiedenen kommerziellen Quellen bezogen und ohne weitere Reinigung eingesetzt. Schmelzpunkte wurden mittels Proben in offenen Kapillaren bestimmt und sind unkorrigiert. ¹H- und ¹³C-NMR-Spektren wurden unter Verwendung eines Bruker-Spektrometers aufgenommen (¹H: 300,13 MHz; ¹³C: 75,5 MHz). Die chemischen Verschiebungen sind in ppm und die Kopplungskonstanten in Hertz (Hz) angegeben. Die Umsätze der aromatischen Substrate wurden mittels HPLC auf einem Shimadzu-Chromatographen unter Verwendung einer Luna-C18-Säule (25 cm x 4,6 mm Ø) mit UV-Detektor bei unterschiedlichen Wellenlängen bestimmt, wobei Umsatzschwankungen um etwa 2 % innerhalb der systematischen Fehlergrenzen liegen. Sämtliche Ergebnisse aller Synthese-, Bezugs- und Ausführungsbeispiele sind Mittelwerte von Dreifachansätzen.

*Probenvorbereitung*: Eine MeCN/H₂O-Lösung (1 ml, 1:1) mit 0,1% TFA wurde zu einer Aliquote des Reaktionsgemischs zugesetzt (1 ml). Das Protein wurde durch Zentrifugation entfernt, und die Lösung wurde durch VIVAspin-Polyethersulfon-Membranfilter filtriert. Die erhaltene Lösung wurde mittels HPLC unter den unten angegebenen Bedingungen analysiert. Die relativen Mengen der Verbindungen wurden aus den jeweiligen Peakflächen berechnet. Säule: Luna-C18, 5 µm; Durchflussrate: 1 ml/min; Temperatur: 30 °C; Gradient: von 100 % H₂O (0,1% TFA) auf 100 % MeCN (0,1% TFA) in 22 min. Wellenlänge: 280 nm.

### Synthesebeispiele 1 bis 3: Herstellung der Enzympräparate

### Synthesebeispiel 1

### Herstellung der TPL als zellfreier Extrakt von Citrobacter freundii M379V

*E.-coli*-Klone, die das M379V- TPL-Plasmid enthielten, wurden in LB-Medium vermehrt, das durch Sterilisieren einer Lösung (1 l) der folgenden Komponenten in 5 1-I-Erlenmeyerkolben hergestellt wurde: Trypton (10 g/l), NaCl (5 g/l) und Hefeextrakt (5 g/l). Eine Vorkultur wurde durch Inokulieren von 100 ml LB-Medium, das Ampicillin (100 mg/l) enthielt, hergestellt. Die Vorkultur wurde über Nacht bei 120 U/min und 37 °C geschüttelt. Danach wurden die Ampicillin (100 mg/l) enthaltenden Kolben mit der Vorkultur inokuliert, was eine anfängliche OD₆₀₀ von 0,05 ergab. Anschließend wurden die Kulturen bei 120 U/min und 30 °C bis zum Erreichen einer OD₆₀₀ von 0,4 bis 0,6 geschüttelt. Die Proteinexpression wurde mit IPTG (0,5 mM, Endkonzentration) induziert, und die Kulturen wurden 2 h lang bei 20 °C und 120 U/min geschüttelt. Abschließend wurden die Zellen durch Zentrifugation (8000 U/min, 20 min) geerntet, mit Kaliumphosphatpuffer (10 mM, pH 7) gewaschen, in KPi-Puffer (50 mM, 180 mM NH₄Cl, 0,04 mM PLP, pH 8) resuspendiert und mittels Ultraschallbehandlung (40 % Amplitude, 1 s Puls an, 2 s Puls aus, 5 min) zerstört. Das Gemisch wurde durch Zentrifugation (15000 U/min, 15 min) geerntet und der Überstand in flüssigem Stickstoff schockgefroren und lyophilisiert. Der lyophilisierte zellfreie Extrakt wurde bei 4 °C aufbewahrt und unverändert in den Reaktionen eingesetzt.

### Synthesebeispiel 2

### Herstellung der TAL von Rhodobacter sphaeroides als Ganzzellenkatalysator

E.-coli-Klone, die das TAL-Plasmid enthielten, wurden in LB-Medium vermehrt, das durch Sterilisieren einer Lösung (1 l) der folgenden Komponenten in 5 1-I-Erlenmeyerkolben hergestellt wurde: Trypton (10 g/l), NaCl (5 g/l) und Hefeextrakt (5 g/l). Eine Vorkultur wurde durch Inokulieren von 100 ml LB-Medium, das Kanamycin (50 mg/l) enthielt, hergestellt. Die Vorkultur wurde über Nacht bei 120 U/min und 37 °C geschüttelt. Danach wurden die Kanamycin (50 mg/l) enthaltenden Kolben mit der Vorkultur inokuliert, was eine anfängliche OD₆₀₀ von 0,05 ergab. Anschließend wurden die Kulturen bei 120 U/min und 37 °C bis zum Erreichen einer OD₆₀₀ von 0,5 bis 0,7 geschüttelt. Die Proteinexpression wurde mit IPTG (0,5 mM, Endkonzentration) induziert, und die Kulturen wurden 24 h lang bei 20 °C und 120 U/min geschüttelt. Abschließend wurden die Zellen durch Zentrifugation (8000 U/min, 20 min) geerntet, mit Kaliumphosphatpuffer (10 mM, pH 8) gewaschen, in flüssigem Stickstoff schockgefroren und lyophilisiert. Die lyophilisierten Zellen wurde bei 4 °C aufbewahrt und unverändert in den Reaktionen eingesetzt.

### Synthesebeispiel 3

### Herstellung der FAD von Enterobacter sp. als E.-coli-Ganzzellenkatalysator

*E.-coli*-Klone, die das FAD-Plasmid enthielten, wurden in LB-Medium vermehrt, das durch Sterilisieren einer Lösung (1 l) der folgenden Komponenten in fünf 1-I-Erlenmeyerkolben hergestellt wurde: Trypton (10 g/l), NaCl (5 g/l) und Hefeextrakt (5 g/l). Eine Vorkultur wurde durch Inokulieren von 100 ml LB-Medium, das Kanamycin (50 mg/l) enthielt, hergestellt. Die Vorkultur wurde über Nacht bei 120 U/min und 37 °C geschüttelt. Danach wurden die Kanamycin (50 mg/l) enthaltenden Kolben mit der Vorkultur inokuliert, was eine anfängliche OD₆₀₀ von 0,05 ergab. Anschließend wurden die Kulturen bei 120 U/min und 37 °C bis zum Erreichen einer OD₆₀₀ von 0,5 bis 0,7 geschüttelt. Die Proteinexpression wurde mit IPTG (0,5 mM, Endkonzentration) induziert, und die Kulturen wurden 24 h lang bei 20 °C und 120 U/min geschüttelt. Abschließend wurden die Zellen durch Zentrifugation (8000 U/min, 20 min) geerntet, mit Kaliumphosphatpuffer (10 mM, pH 8) gewaschen, in flüssigem Stickstoff schockgefroren und lyophilisiert. Die lyophilisierten Zellen wurde bei 4 °C aufbewahrt und unverändert in den Reaktionen eingesetzt.

### Bezugsbeispiele 1 bis 3: Aktivitätsmessungen

### Bezugsbeispiel 1

### Aktivität der TPL

Zum besseren Vergleich der Enzymaktivität wurde die Aktivität der TPL bei der C-C-Kupplung zwischen Phenol **1**, Brenztraubensäure bzw. Pyruvat und Ammoniak gemäß Schritt a) durch Messung der anfänglichen Reaktionsgeschwindigkeit mittels HPLC bestimmt. Eine Aktivitätseinheit wurde als jene Menge an Katalysator definiert, die die Bildung von 1 µMol Tyrosin pro Minute unter den folgenden Bedingungen katalysierte: 30 °C, KPi-Puffer 50 mM, pH 8, 850 U/min. Das Testgemisch enthielt 2-Chlorphenol (**1b**, 23 mM), Pyruvat (46 mM), NH₄Cl (180 mM) und den gefriergetrockneten zellfreien Extrakt aus Synthesebeispiel 1, der die überexprimierte TPL enthielt (2 mg). Die Reaktionen wurden durch den Zusatz des Enzyms gestartet, und der Umsatz wurde zwischen 1 und 10 min bestimmt. Alle Messungen wurden in zumindest drei Ansätzen durchgeführt. Die festgestellten Reaktionsaktivitäten entsprachen 0,30 U pro mg zellfreier Extrakt.

### Bezugsbeispiel 2

### Aktivität der TAL

Zum besseren Vergleich der Enzymaktivität wurde die Aktivität der TAL bei der Umsetzung des Tyrosins **2** zum Zimtsäurederivat **3** gemäß Schritt b) durch Messung der anfänglichen Reaktionsgeschwindigkeit mittels HPLC bestimmt. Eine Aktivitätseinheit wurde als jene Menge an Katalysator definiert, die die Bildung von 1 µMol Zimtsäure **3** pro Minute unter den folgenden Bedingungen katalysierte: 30 °C, KPi-Puffer 50 mM, pH 8, 850 U/min. Das Testgemisch enthielt L-3-Chlortyrosin (**2b**, 5 mM), NH₄Cl (180 mM) und *E*.-*coli*-Ganzzellen aus Synthesebeispiel 2, die die TAL überexprimierten (5 mg). Die Reaktionen wurden durch den Zusatz des Enzyms gestartet, und der Umsatz wurde zwischen 5 und 20 min bestimmt. Alle Messungen wurden in zumindest drei Ansätzen durchgeführt. Die festgestellten Reaktionsaktivitäten entsprachen 9,2 mU pro mg *E*.-*coli*-Ganzzellen.

### Bezugsbeispiel 3

### Aktivität der FAD

Zum besseren Vergleich der Enzymaktivität wurde die Aktivität der FAD bei der Decarboxylierung des Zimtsäurederivats **3** zum Vinylphenol **4** gemäß Schritt c) durch Messung der anfänglichen Reaktionsgeschwindigkeit mittels HPLC bestimmt. Eine Aktivitätseinheit wurde als jene Menge an Katalysator definiert, die die Bildung von 1 µMol Vinylphenol **4** pro Minute unter den folgenden Bedingungen katalysierte: 30 °C, KPi-Puffer 50 mM, pH 8, 850 U/min. Das Testgemisch enthielt 3-Chlor-4-hydroxy-zimtsäure (3-Chlorcumarsäure, **3b**, 5 mM), NH₄Cl (180 mM) und *E*.-*coli*-Ganzzellen aus Synthesebeispiel 3, die die FAD überexprimierten (0,1 mg). Die Reaktionen wurden durch den Zusatz des Enzyms gestartet, und der Umsatz wurde zwischen 1 und 5 min bestimmt. Alle Messungen wurden in zumindest drei Ansätzen durchgeführt. Die festgestellten Reaktionsaktivitäten entsprachen 2,1 U pro mg *E*.-*coli*-Ganzzellen.

### Bezugsbeispiele 4 bis 6: Bestimmung des pH-Optimums

### Bezugsbeispiel 4

### pH-Optimum der TPL

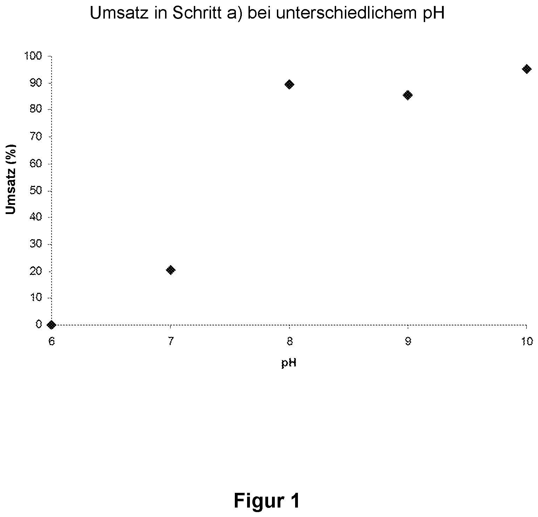

Die TPL-katalysierte enzymatische Reaktion aus Bezugsbeispiel 1 zwischen 2-Chlorphenol **1b** (23 mM), Pyruvat (46 mM) und NH₄⁺ (180 mM) unter Verwendung des gefriergetrockneten zellfreien Extrakts aus Synthesebeispiel 1 wurde mit Zusatz von 0,4 mM Pyridoxalphosphat (PLP) als Coenzym unter Variation des pH wiederholt und 22 Stunden lang ablaufen gelassen. Fig. 1 zeigt die Ergebnisse für pH-Werte von 6 bis 10, aus denen hervorgeht, dass die besten Umsätze zum L-3-Chlortyrosin **2b** bei stark alkalischem pH erzielt werden, da in diesem Bereich ungeladener Ammoniak an der Reaktion teilnehmen kann. Interessanterweise bleibt das Enzym selbst im stark alkalischen pH-Bereich stabil, zumal bei pH 10 sogar der beste Umsatz erzielt wurde und das Optimum daher wohl noch darüber liegen wird.

### Bezugsbeispiel 5

### pH-Optimum der TAL

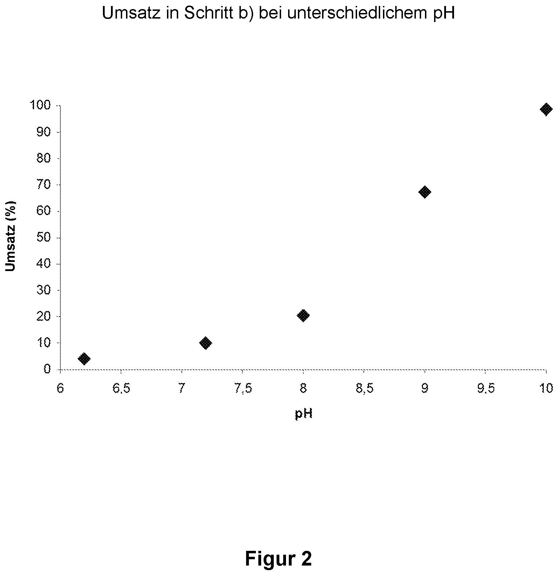

Die TAL-katalysierte enzymatische Reaktion aus Bezugsbeispiel 2 zwischen 3-Chlortyrosin **2b** (10 mM) und NH₄⁺ (180 mM) unter Verwendung des Ganzzellpräparats aus Synthesebeispiel 2 wurde ebenfalls unter Zusatz von 0,4 mM Pyridoxalphosphat (PLP) als Coenzym und Variation des pH wiederholt und 6 Stunden lang ablaufen gelassen. Fig. 2 zeigt wiederum die Ergebnisse für pH-Werte von 6 bis 10, aus denen erneut hervorgeht, dass der stark alkalische pH-Bereich über pH 10 zu bevorzugen ist. In diesem Fall wird das Reaktionsoptimum mit Sicherheit über pH 10 liegen, wie dies durch Extrapolation einer durch die Messpunkte gelegten gedanklichen Ausgleichskurve nahe gelegt wird.

### Bezugsbeispiel 6

### pH-Optimum der FAD

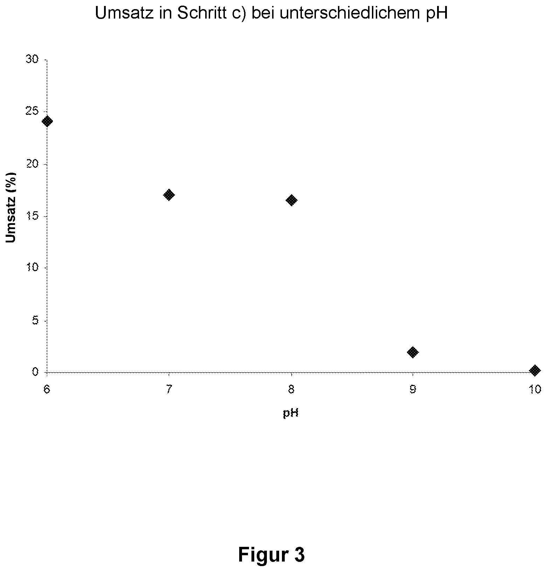

Die FAD-katalysierte enzymatische Reaktion aus Bezugsbeispiel 3, d.h. die Decarboxylierung von 3-Chlorcumarsäure **3b** (10 mM) unter Verwendung des Ganzzellpräparats aus Synthesebeispiel 3 zum 4-Vinylphenol **4b** wurde wiederum unter Zusatz von 0,4 mM Pyridoxalphosphat (PLP) als Coenzym und Variation des pH wiederholt und 1 Stunde lang ablaufen gelassen. Auch Fig. 3 zeigt die Ergebnisse für pH-Werte von 6 bis 10, aus denen zu erkennen ist, dass bis pH 8 vergleichbare Umsätze wie im neutralen Bereich erzielbar sind, während es bei pH 9 kaum noch zu Umsatz kam und bei pH 10 praktisch kein Umsatz mehr zu erkennen ist. In diesem Fall wird das Reaktionsoptimum mit Sicherheit im sauren pH-Bereich liegen, wie dies eine Extrapolation der gedanklichen Ausgleichskurve nahe legt und für Decarboxylierungsreaktionen für den Fachmann auch zu erwarten war.

### Beispiele 1 bis 4

### Eintopfreaktion der Schritte a) bis c) bei variierendem pH

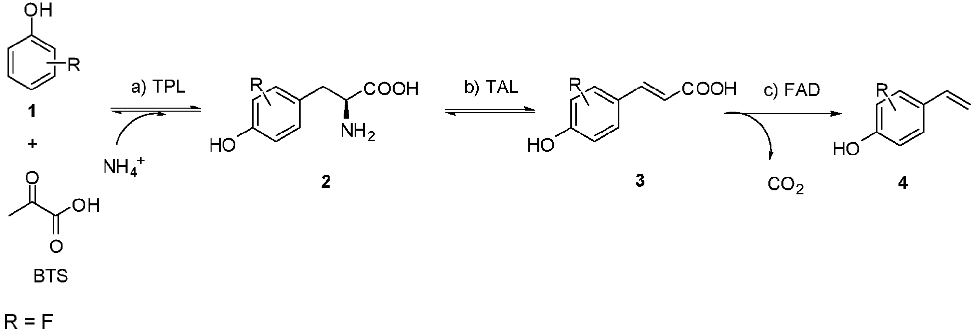

Unter Verwendung der drei Enzyme aus den Synthesebeispielen 1 bis 3 wurde die dreistufige Eintopfreaktion mit 2-Fluorphenol **1a** (R = F) unter den folgenden Bedingungen durchgeführt: **1a** (23 mM); BTS (Pyruvat) (46 mM); TPL (4 mg); TAL (20 mg); FAD (5 mg); wässriger Puffer (50 mM): KPi für pH 7-8, CHES für pH 9-10; Zeit: 6 h; 20 °C; Schütteln bei 850 U/min. Der pH wurde zwischen 7 und 10 variiert, und es wurden in zeitlichen Intervallen Proben gezogen und mittels HPLC auf den Gehalt an 2-Fluor-4-vinylphenol **4a** (R = F) untersucht.

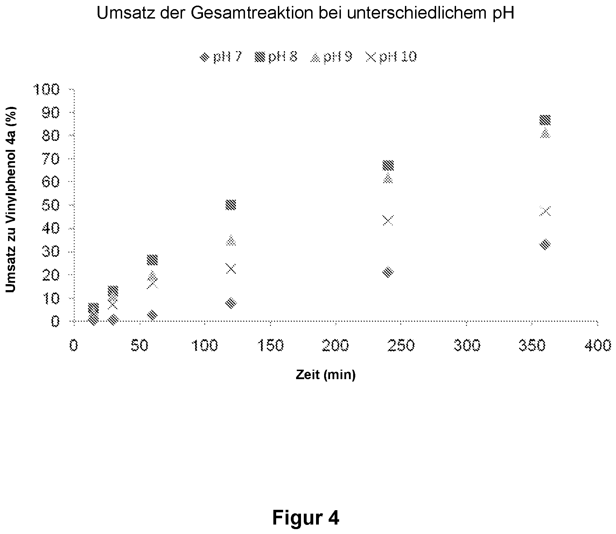

Fig. 4 zeigt die Ergebnisse dieser Reaktionen. Es zeigte sich, dass die Umsätze bei pH 7 (Beispiel 1; ca. 30 %) bzw. pH 10 (Beispiel 4; ca. 45 %) deutlich unter jenen bei pH 8 (Beispiel 2; ca. 85 %) und pH 9 (Beispiel 3; ca. 80 %) lagen, die ihrerseits nahezu gleiche Umsätze ergaben, was einerseits auf die geringe Aktivität zweier der Enzyme, nämlich TPL und TAL, bei pH 7 (siehe die Fig. 1 und 2) und andererseits auf die nahezu Unwirksamkeit der FAD bei pH 10 (siehe Fig. 3) zurückgeführt werden kann. In Anbetracht Letzterer war es überraschend, dass bei pH 10 dennoch signifikant bessere Umsätze erzielbar sind als bei pH 7.

Zur Untersuchung dieses Phänomens wurden die Beispiele 1 und 4 bei pH 7 bzw. pH 10 wiederholt und dabei in zeitlichen Intervallen die Mengen des Edukts **1a** und der Produkte **2a** bis **4a** mittels HPLC bestimmt.

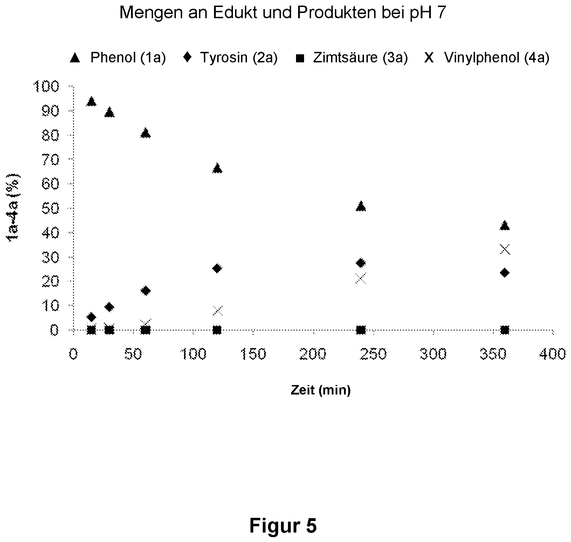

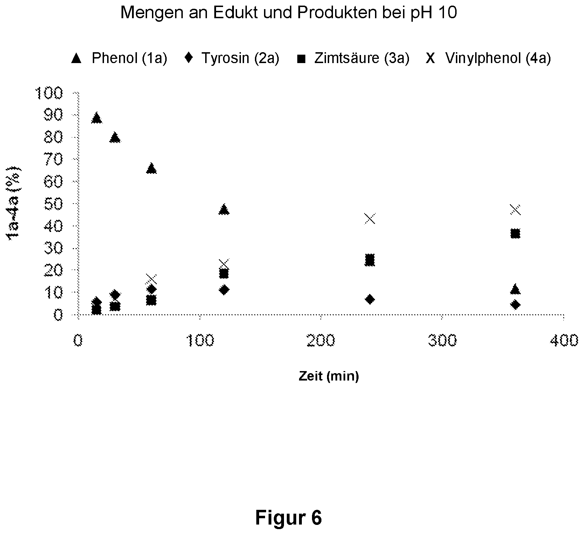

Die Fig. 5 und 6 zeigen die Ergebnisse dieser Versuche für das Phenol 1a (▲), das Tyrosin 2a (◆), die Zimtsäure 3a (■) und das gewünschte Vinylphenol **4a** (x). Dies bestätigte die obigen Vermutungen, da sich zeigte, dass bei pH 7 die Menge an Edukt **1a** nur sehr langsam abnimmt und auch nach 6 h noch über 40 % des Ausgangsphenols vorhanden sind, während die gesamte Zeit über praktisch keine Zimtsäure im Reaktionsgemisch enthalten ist. Somit ist bei pH 7 Schritt a) die geschwindigkeitsbestimmende Teilreaktion, während die Decarboxylierung von Schritt c) am raschesten abläuft. Bei pH 10 hingegen erfolgt die C-C-Kupplung von Schritt a) deutlich rascher, so dass nach 6 h das Ausgangsprodukt **1a** auf 10 % der ursprünglichen Menge abgenommen hatte. Gleichzeitig sammelt sich die Zimtsäure **3a** jedoch stetig im Reaktionsgemisch an, was bedeutet, dass bei pH 10 die Decarboxylierung die langsamste und damit die geschwindigkeitsbestimmende Teilreaktion darstellt.

Aufgrund der Notwendigkeit, zur Einstellung von pH 9 CHES- oder andere, deutlich toxischere wässrige Puffer als den KPi-Puffer für pH 8 verwenden zu müssen, wurde - trotz etwa gleicher Umsätze - von den Erfindern pH 8 als optimaler pH für die Durchführung des erfindungsgemäßen Verfahrens angesehen.

### Beispiele 5 bis 8

### Eintopfreaktion bei pH 8 mit Co-Lösungsmittel

Das obige Beispiel 2 bei pH 8 wurde daher wiederholt, wobei variierende Mengen an Co-Lösungsmitteln zugemischt und die Mengen des Endprodukts, p-Vinylphenol **4a**, bestimmt wurden. In Beispiel 5 wurde zu Vergleichszwecken Beispiel 2 ohne Co-Lösungsmittel wiederholt, während in Beispiel 6 DMSO als Vertreter eines wassermischbaren Lösungsmittels (5 Vol.-%), in Beispiel 7 Diethylether als Vertreter eines mit Wasser nicht mischbaren Lösungsmittels (5 Vol.-%) und in Beispiel 8 die doppelte Menge an Diethylether (10 Vol.-%) zugesetzt wurden, jeweils bezogen auf das Volumen der wässrigen Pufferlösung.

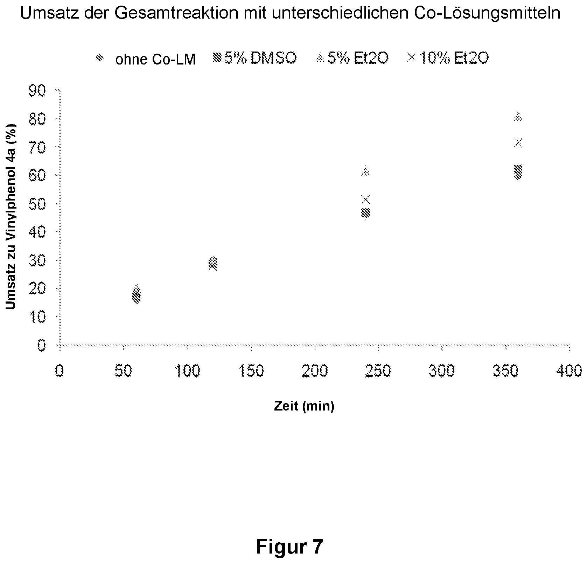

Fig. 7 zeigt die Ergebnisse dieser Versuche. Es ist zu erkennen, dass die Gegenwart von wassermischbarem DMSO für den Umsatz zum Produkt **4a** praktisch keinen Unterschied machte, während mit Wasser nicht mischbarer Diethylether nach 6 h Reaktionszeit in einer Menge von 10 Vol.-% eine Umsatzsteigerung von über 10 Prozentpunkten, in einer Menge von 5 Vol.-% aber sogar von über 20 Prozentpunkten bewirkte.

Ohne sich auf eine bestimmte Theorie festlegen zu wollen, nehmen die Erfinder an, dass die Umsatzsteigerungen auf die Ausbildung zweier Phasen zurückzuführen ist, wobei gewisse Anteile der vier verschiedenen Phenole - aufgrund ihrer geringeren Polarität und Hydrophilie anzunehmenderweise vorwiegend Anteile des Edukts **1a** und des Endprodukts **4a** - in der Etherphase gelöst werden. Dadurch wird eine etwaige Inhibierung der Einzelreaktionen - vermutlich der Schritte b) und c) - unterdrückt und/oder das chemische Gleichgewicht der Gesamtreaktion noch weiter nach rechts verschoben. Für eine Unterdrückung von inhibierenden Effekten spricht, dass mit der doppelten Menge an Ether keine so starke Umsatzsteigerung erzielbar ist, da dann offenbar zu große Mengen der Phenole - möglicherweise des Edukts - in der organischen Phase gelöst sind, was die Reaktionsgeschwindigkeit bremst.

### Beispiel 9

### Eintopfreaktion unter optimalen Bedingungen

Nach somit erfolgter Optimierung von pH und Co-Lösungsmittel wurde der Versuch aus Beispiel 7 unter den folgenden Bedingungen wiederholt: **1a** (23 mM); Pyruvat (46 mM); TPL (4 mg); TAL (20 mg); FAD (5 mg); KPi-Puffer (50 mM), pH 8; 5 Vol.-% Et₂O, Zeit: 6 h; 30 °C; Schütteln bei 850 U/min; und dabei in zeitlichen Intervallen die Mengen des Edukts **1a** und der Produkte **2a** bis **4a** mittels HPLC bestimmt.

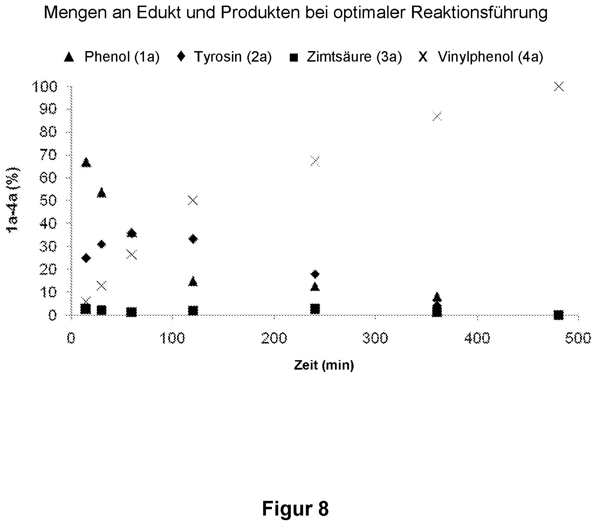

Fig. 8 zeigt das Ergebnis dieses Versuchs, wobei vor allem die außerordentlich rasche Abnahme der Menge an Edukt **1a** während der ersten Minuten sowie der nach 6 h erzielte Umsatz zum Endprodukt **4a** von über 95 % auffallen.

### Beispiele 10 bis 20

### Herstellung verschiedener Derivate

Unter Anwendung bzw. geringfügiger Variation dieser optimalen Reaktionsbedingungen aus Beispiel 9 wurden das Substitutionsmuster (und mitunter auch die Menge des Ausgangssubstrats, des Phenols **1**) wie in nachstehender Tabelle 1 angegeben variiert, und die Umsätze, d.h. die relativen Mengen nach Beendigung der Reaktion, wurden nach jeweils 24 h bzw. 48 h mittels HPLC bestimmt und sind ebenfalls in der Tabelle angeführt.

**Tabelle 1**

| Beispiel | Phenol **1** | R | Konz. 1 [mM] | Menge 1 [%] | Menge 2 [%] | Menge 3 [%] | Menge 4 [%] |
|---|---|---|---|---|---|---|---|
| 10 | **1a** | 2-Fluor | 23 | < 1 | < 1 | < 1 | > 97 |
| 11 | **1a** | 2-Fluor | 46^{a} | < 1 | < 1 | < 1 | > 97 |
| 12 | **1b** | 2-Chlor | 23 | < 1 | < 1 | < 1 | > 97 |
| 13 | **1c** | 2-Brom | 23 | < 1 | < 1 | < 1 | > 97 |
| 14 | **1d** | 2-Methyl | 23 | < 1 | < 1 | < 1 | > 97 |
| 15 | **1e** | 3-Fluor | 23 | < 1 | < 1 | < 1 | > 97 |
| 16 | **1e** | 3-Fluor | 46^{a,b} | < 1 | < 1 | < 1 | > 97 |
| 17 | **1f** | 3-Chlor | 23 | < 1 | < 1 | < 1 | > 97 |
| 18 | **1g** | 2,3-Difluor | 23 | < 1 | < 1 | < 1 | > 97 |
| 19 | **1g** | 2,3-Difluor | 46^{a,b} | < 1 | < 1 | < 1 | > 97 |
| 20 | **1h** | 2-Fluor-3-chlor | 23^{a} | < 1 | < 1 | < 1 | > 97 |

| | | | | | | | |
|---|---|---|---|---|---|---|---|
| Anm. a: Doppelte Menge an TAL Anm. b: Doppelte Reaktionszeit (48 h) | | | | | | | |

Es zeigte sich, dass unabhängig von der Art (elektonenziehend oder -schiebend) und der Anzahl (einer oder zwei) der Substituenten R stets Umsätze zum jeweiligen Produkt **4** von über 97 % erzielt wurden und die Edukte der drei Teilreaktionen jeweils zu weniger als 1 % enthalten waren.

Der Fachmann darf davon ausgehen, dass die Reaktion auch mit anderen Substitutionsmustern am Ausgangsphenol **1** in ähnlich vollständiger Weise ablaufen wird, solange die Substituenten die enzymatischen Reaktionen nicht signifikant behindern.

### Aufarbeitung:

Die Reaktionen wurden durch Zugabe von gesättigter wässriger NH₄Cl-Lösung gestoppt und mit Ethylacetat (3 × 15 ml) extrahiert. Die vereinigten organischen Phasen wurden über Na₂SO₄ getrocknet und unter reduziertem Druck eingedampft. Der Rückstand wurde mittels Flash-Chromatographie (20% EtOAc/Hexan) gereinigt, und die das jeweilige Vinylphenol enthaltenden Fraktionen wurden vereinigt und bei 100 mmHg eingedampft, was die gewünschten *p*-Vinylphenole durchwegs als farblose Öle in Ausbeuten von 80-90 % d. Th. ergab. Die spektroskopischen Daten der so hergestellten p-Vinylphenole sind nachstehend angegeben.

### 2-Fluor-4-vinylphenol 4a:

¹H-NMR (300 MHz, MeOD): δ_{H} [ppm] = 5,10 (d, ³*J*_{2'c,1'} = 10,9 Hz, 1 H, 2'-H_{c}), 5,59 (dd, ²*J*_{2't,2'c} = 0,8 Hz, ³*J*_{2't,1'} = 17,6 Hz, 1 H, 2'-Hₜ), 6,59 (dd, ³*J*_{1',2'c} = 10,9 Hz, ³*J*_{1',2't} = 17,6 Hz, 1 H, 1'-H), 6,85 (dd, ⁴*J*_{6,F} = 8,8 Hz, ³*J*_{6,5} = 8,5 Hz, 1 H, 6-H), 7,02 (dd, ⁴*J*_{5,3} = 2,3 Hz, ³*J*_{5,6} = 8,4 Hz, 1 H, 5-H), 7,14 (dd, ⁴*J*_{3,5} = 2,1 Hz, ³*J*_{3,F} = 12,4 Hz, 1 H, 3-H).
¹³C-NMR (75 MHz, MeOD): δ_{C} [ppm] = 112,3 (C-2'), 114,1 (d, ²*J*_{3,F} = 18,9 Hz, C-3), 118,5 (d, ³*J*_{6,F} = 3,1 Hz, C-6), 123,7 (d, ⁴*J*_{5,F} = 3,1 Hz, C-5), 131,6 (d, ³*J*_{4,F} = 6,2 Hz, C-4), 136,9 (C-1'), 145,9 (d, ²*J*_{1,F} = 13,3 Hz, C-1), 152 (d, ¹*J*_{2,F} = 240,1 Hz, C-2). ¹⁹F (282 MHz, MeOD)δ_{F} [ppm] = -139,7 (dd, ⁴*J*_{F,6} = 9,1 Hz, ⁴*J*_{F,3} = 12,5 Hz).

### 2-Chlor-4-vinylphenol 4b:

¹H-NMR (300 MHz, MeOD): δ_{H} [ppm] = 5,06 (dd, ²*J*_{2'c,2't} = 0,7 Hz, ³*J*_{2'c,1'} = 10,9 Hz, 1 H, 2'-H_{c}), 5,56 (dd, ²*J*_{2't,2'c} = 0,8 Hz, ³*J*_{2't,1'} = 17,6 Hz, 1 H, 2'-Hₜ), 6,55 (dd, ³*J*_{1',2'c} = 10,9 Hz, ³*J*_{1',2't} = 17,6 Hz, 1 H, 1'-H), 6,81 (d, ³*J*_{6,5} = 8,4 Hz, 1 H, 6-H), 7,15 (dd, ⁴*J*_{5,3} = 2,2 Hz, ³*J*_{5,6} = 8,4 Hz, 1 H, 5-H), 7,32 (d, ⁴*J*_{3,5} = 2,2 Hz, 1 H, 3-H).
¹³C-NMR (75 MHz, MeOD): δ_{C} [ppm] = 112,4 (C-2'), 117,5 (C-6), 121,8 (C-2), 126,8 (C-3), 128,6 (C-5), 132,0 (C-4), 136,7 (C-1'), 154 (C-1).

### 2-Brom-4-vinylphenol 4c:

¹H-NMR (300 MHz, MeOD): δ_{H} [ppm] = 5,07 (dd, ²*J*_{2'c,2't} = 0,6 Hz, ³*J*_{2'c,1'} = 11,0 Hz, 1 H, 2'-H_{c}), 5,57 (dd, ²*J*_{2't,2'c} = 0,6 Hz, ³*J*_{2't,1'} = 17,6 Hz, 1 H, 2'-Hₜ), 6,55 (dd, ³*J*_{1',2'c} = 10,9 Hz, ³*J*_{1',2't} = 17,6 Hz, 1 H, 1'-H), 6,84 (d, ³*J*_{6,5} = 8,4 Hz, 1 H, 6-H), 7,21 (dd, ⁴*J*_{5,3} = 2,1 Hz, ³*J*_{5,6} = 8,4 Hz, 1 H, 5-H), 7,51 (d, ⁴*J*_{3,5} = 2,1 Hz, 1 H, 3-H).
¹³C-NMR (75 MHz, MeOD): δ_{C} [ppm] = 110,9 (C-2), 112,4 (C-2'). 117,1 (C-6), 127,4 (C-5), 131,7 (C-3), 132,3 (C-4), 136,4 (C-1'), 154,9 (C-1).

### 2-Methyl-4-vinylphenol 4d:

¹H-NMR (300 MHz, MeOD): δ_{H} [ppm] = 2,17 (s, 3 H, CH₃), 4,99 (dd, ²*J*_{2'c,2't} = 1,1 Hz, ³*J*_{2'c,1'} = 10,9 Hz, 1 H, 2'-H_{c}), 5,53 (dd, ²*J*_{2't,2'c} = 1,1 Hz, ³*J*_{2't,1'} = 17,7 Hz, 1 H, 2'-Hₜ), 6,58 (dd, ³*J*_{1',2'c} = 10,9 Hz, ³*J*_{1',2't} = 17,6 Hz, 1 H, 1'-H), 6,68 (d, ³*J*_{6,5} = 8,2 Hz, 1 H, 6-H), 7,06 (dd, ⁴*J*_{5,3} = 2,2 Hz, ³*J*_{5,6} = 8,2 Hz, 1 H, 5-H), 7,13 (d, ⁴*J*_{3,5} = 2,1 Hz, 1 H, 3-H).
¹³C-NMR (75 MHz, MeOD): δ_{C} [ppm] = 16,2 (CH₃ an C-2), 110,4 (C-2'), 115,5 (C-6), 125,5 (C-2), 125,8 (C-5), 129,6 (C-3), 130,6 (C-4), 138,1 (C-1'), 156,5 (C-1).

### 3-Fluor-4-vinylphenol 4e:

¹H-NMR (300 MHz, MeOD): δ_{H} [ppm] = 5,16 (dd, ²*J*_{2'c,2't} = 1,4 Hz, ³*J*_{2'c,1'} = 11. 3 Hz, 1 H, 2'-H_{c}), 5,64 (dd, ²*J*_{2't,2'c} = 1,3 Hz, ³*J*_{2't,1'} = 17,8 Hz, 1 H, 2'-Hₜ), 6,47 (dd, ⁴*J*_{2,6} = 2,4 Hz, ³*J*_{2,F} = 12,5 Hz, 1 H, 2-H), 6,57 (dd, ⁴*J*_{6,2} = 2,5 Hz, ³*J*_{6,5} = 8,6 Hz, 1 H, 6-H), 6,74 (dd, ³*J*_{1',2'c} = 11,3 Hz, ³*J*_{1',2't} = 17,8 Hz, 1 H, 1'-H), 7,35 (dd, ³*J*_{5,6} = 8,7 Hz, ⁴*J*_{5,F} = 8,8 Hz, 1 H, 5-H).
¹³C-NMR (75 MHz, MeOD): δ_{C} [ppm] = 103,5 (d, ²*J*_{2,F} = 25,0 Hz, C-2), 112,6 (d, ⁴*J*_{2',F} = 2,7 Hz, C-2'), 113,4 (d, ⁴*J*_{6,F} = 4,8 Hz, C-6), 117,8 (d, ³*J*_{4,F} = 12,6 Hz, C-4), 128,8 (d, ³*J*_{1',F} = 5,8 Hz, C-1'), 130,3 (d, ³*J*_{5,F} = 3,5 Hz, C-5), 159,9 (d, ²*J*_{2,F} = 11,9 Hz, C-1), 162,3 (d, ¹*J*_{3,F} = 247,4 Hz, C-3); ¹⁹F (282 MHz, MeOD): δ_{F} [ppm] = -119,8 (dd, ⁴*J*_{F,5} = 8,8 Hz, ³*J*_{F,2} = 12,6 Hz).

### 3-Chlor-4-vinylphenol 4f:

¹H-NMR (300 MHz, MeOD): δ_{H} [ppm] = 5,18 (dd, ²*J*_{2'c,2't} = 1,2 Hz, ³*J*_{2'c,1'} = 11,0 Hz, 1 H, 2'-H_{c}), 5,60 (dd, ²*J*_{2't,2'c} = 1,2 Hz, ³*J*_{2't,1'} = 17,5 Hz, 1 H, 2'-Hₜ), 6,71 (dd, ⁴*J*_{6,2} = 2,5 Hz, ³*J*_{6,5} = 8,6 Hz, 1 H, 6-H), 6,79 (d, ³*J*_{2,6} = 2,5 Hz, 1 H, 2-H), 6,98 (dd, ³*J*_{1',2'c} = 11,0 Hz, ³*J*_{1',2't} = 17,6 Hz, 1 H, 1'-H), 7,47 (d, ³*J*_{5,6} = 8,6 Hz, 1 H, 5-H).
¹³C-NMR (75 MHz, MeOD): δ_{C} [ppm] = 113,8 (C-2'), 115,7 (C-6), 116,8 (C-2), 128,1 (C-4), 128,3 (C-5), 133,7 (C-1'), 134,4 (C-3), 159,2 (C-1).

### 2,3-Difluor-4-vinylphenol 4g:

¹H-NMR (300 MHz, MeOD): δ_{H} [ppm] = 5,25 (dd, ²*J*_{2'c,2't} = 1,1 Hz, ³*J*_{2'c,1'} = 11,4 Hz, 1 H, 2'-H_{c}), 5,70 (dd, ²*J*_{2't,2'c} = 1,1 Hz, ³*J*_{2't,1'} = 17,8 Hz, 1 H, 2'-Hₜ), 6,68 (ddd, ⁵*J*_{6,3F} = 2,0 Hz, ⁴*J*_{6,2F} = 8,0 Hz, ³*J*_{6,5} = 8,5 Hz, 1 H, 6-H), 6,72 (dd, ³*J*_{1',2'c} = 11,3 Hz, ³*J*_{1',2't} = 17,8 Hz, 1 H, 1'-H), 7,11 (ddd, 5*J*_{5,2F} = 2,3 Hz, ⁴*J*_{5,3F} = 8,3 Hz, ³*J*_{5,6} = 8,5 Hz, 1 H, 5-H).
¹³C-NMR (75 MHz, MeOD): δ_{C} [ppm] =113,7 (dd, ³*J*_{6,2F} = 3,2 Hz, ⁴*J*_{6,3F} = 3,2 Hz, C-6), 115,1 (d, ⁴J_{*2'*,3F} = 5,1 Hz, C-2'), 119,2 (dd, ³*J*_{4,2F} = 1,1 Hz, ²*J*_{4,3F} = 9,7 Hz, C-4), 122,2 (dd, ³*J*_{5,3F} = 4,5 Hz, ⁴*J*_{5,2F} = 4,5 Hz, C-5), 129,6 (dd, ⁴*J*_{1',2F} = 3,1 Hz, ³*J*_{1',3F} = 3,3 Hz, C-1'), 141,8 (dd, ²*J*_{2,3F} = 14,5 Hz, ¹*J*_{2,2F} = 241,2 Hz, C-2), 147,5 (dd, ³*J*_{1,3F} = 2,8 Hz, ²*J*_{1,2F} = 10,2 Hz, C-1), 150,7 (dd, ²*J*_{3,2F} = 10,9 Hz, ¹*J*_{3,3F} = 248,4 Hz, C-3); ¹⁹F (282 MHz, MeOD): δ_{F} [ppm] = -146,1 (ddd, ⁵*J*_{3F,6} = 2,0 Hz, ⁴*J*_{3F,5} = 8,1 Hz, ³*J*_{3F,2F} = 18,4 Hz, 1 F, F an C-3), -165,1 (ddd, ⁵*J*_{2F,5} = 2,4 H, ⁴*J*_{2F,6} = 8,0 Hz, ³*J*_{2F,3F} = 18,3 Hz, 1 F, F an C-2).

### 3-Chlor-2-fluor-4-vinylphenol 4h:

¹H-NMR (300 MHz, MeOD): δ_{H} [ppm] = 5,27 (d, ³*J*_{2'c,1'} = 11,1 Hz, 1 H, 2'-H_{c}), 5,68 (dd, ²*J*_{2't,2'c} = 0,9 Hz, ³*J*_{2't,1'} = 17,5 Hz, 1 H, 2'-Hₜ), 6,85 (dd, ⁴*J*_{6,F} = 8,6 Hz, ³*J*_{6,5} = 8,6 Hz, 1 H, 6-H), 6,96 (dd, ³*J*_{1',2'c} = 11,0 Hz, ³*J*_{1',2't} = 17,5 Hz, 1 H, 1'-H), 7,29 (dd, ⁵*J*_{5,F} = 2,0 Hz, ³*J*_{5,6} = 8,7 Hz, 1 H, 5-H).
¹³C-NMR (75 MHz, MeOD): δ_{C} [ppm] = 115,4 (C-2'), 117,1 (d, ³*J*_{6,F} = 3,1 Hz, C-6), 121,5 (d, ²*J*_{3,F} = 15,1 Hz, C-3), 122,2 (d, ⁴*J*_{5,F} = 4,0 Hz, C-5), 129,2 (d, ³*J*_{4,F} = 1,3 Hz, C-4), 132,9 (d, ⁴*J*_{1',F} = 3,1 Hz, C-1'), 146,8 (d, ²*J*_{1,F} = 13,3 Hz, C-1), 148,9 (d, ¹*J*_{2,F} = 241,2 Hz, C-2); ¹⁹F (282 MHz, MeOD): δ_{F} [ppm] = - 139,6 (dd, ⁴*J*_{F,6} = 8,8 Hz, ⁵*J*_{F,5} = 2,0 Hz).

Die vorliegende Erfindung stellt somit ein Eintopfverfahren zur Herstellung von *p-*Vinylphenolen bereit, nach dem eine Vielzahl von Verbindungen effizient und kostengünstig hergestellt werden kann, was nach dem Stand der Technik nicht einmal annähernd möglich war.

## Patentansprüche

1. Biokatalytisches Verfahren zur Herstellung von *p*-Vinylphenolen, umfassend eine dreistufige Eintopfreaktion gemäß nachstehendem Reaktionsschema: 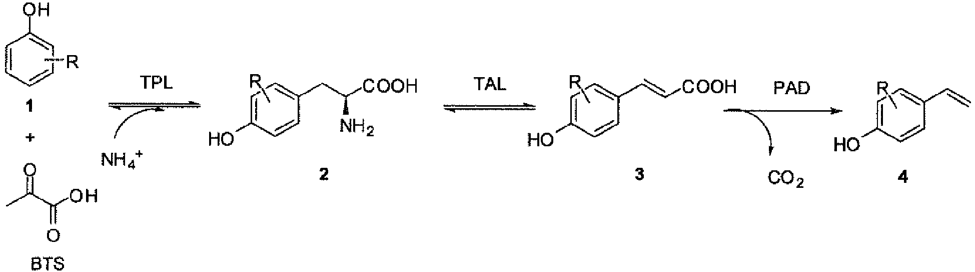 wobei
a) ein gegebenenfalls substituiertes Phenol **1** mittels katalytischer Wirkung einer Tyrosin-Phenol-Lyase (TPL) und in Gegenwart von Ammoniumionen mit Brenztraubensäure (BTS) zum gegebenenfalls substituierten Tyrosin **2** verbunden wird,
b) aus dem Tyrosin **2** mittels katalytischer Wirkung einer Tyrosin-Ammoniak-Lyase (TAL) Ammoniak eliminiert wird, um eine gegebenenfalls substituierte p-Cumarsäure **3** zu ergeben, und
c) die p-Cumarsäure **3** mittels katalytischer Wirkung einer Phenolsäure-Decarboxylase (PAD) einer Decarboxylierung unterzogen wird, um das gewünschte, gegebenenfalls substituierte *p*-Vinylphenol **4** zu ergeben;
d) wobei das entstehende CO₂ aus dem Reaktionssystem entfernt wird, um das chemische Gleichgewicht aller drei Reaktionsschritte in Richtung der Produkte zu verschieben.

2. Verfahren nach Anspruch 1, **dadurch gekennzeichnet, dass** die Reaktion bei einem pH von etwa 8 bis 9 durchgeführt wird.

3. Verfahren nach Anspruch 2, **dadurch gekennzeichnet, dass** die Reaktion bei einem pH von etwa 8 durchgeführt wird.

4. Verfahren nach einem der Ansprüche 1 bis 3, **dadurch gekennzeichnet, dass** in Schritt b) die Tyrosin-Ammoniak-Lyase (TAL) in Form von Ganzzellen, die das rekombinante Enzym enthalten, eingesetzt wird.

5. Verfahren nach einem der Ansprüche 1 bis 4, **dadurch gekennzeichnet, dass** in Schritt c) eine Ferulasäure-Decarboxylase (FAD) als Katalysator eingesetzt wird.

6. Verfahren nach Anspruch 5, **dadurch gekennzeichnet, dass** die Ferulasäure-Decarboxylase (FAD) in Form von Ganzzellen, die das rekombinante Enzym enthalten, eingesetzt wird.

7. Verfahren nach einem der Ansprüche 1 bis 6, **dadurch gekennzeichnet, dass** die Eintopfreaktion in einem wässrigen Puffersystem in Gegenwart eines mit Wasser nicht mischbaren Co-Lösungsmittels durchgeführt wird.

8. Verfahren nach Anspruch 7, **dadurch gekennzeichnet, dass** Diethylether als Co-Lösungsmittel, vorzugsweise in einer Menge von 5 Vol.-%, bezogen auf das wässrige Puffersystem, eingesetzt wird.

9. Verfahren nach einem der vorangegangenen Ansprüche, **dadurch gekennzeichnet, dass** das Phenol 1 in ortho- und/oder meta-Stellung mit einem oder mehreren Substituenten R substituiert ist, die aus Halogenen, C₁₋₆-Alkyl und C₁₋₆-Alkoxy ausgewählt sind.

## Claims

1. A biocatalytic method for preparing p-vinyl phenols, comprising a three-step one-pot reaction according to the following reaction scheme: 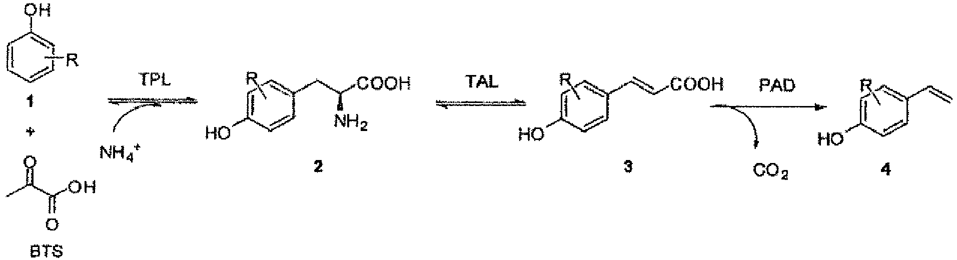 wherein
a) an optionally substituted phenol **1** is bound to pyruvic acid (BTS) by means of the catalytic action of a tyrosine phenol-lyase (TPL) and in the presence of ammonium ions to give an optionally substituted tyrosine **2**,
b) ammonia is eliminated from the tyrosine **2** by means of the catalytic action of a tyrosine ammonia-lyase (TAL) to give an optionally substituted *p*-coumaric acid **3**, and
c) the *p*-coumaric acid **3** is subjected to a decarboxylation reaction by means of the catalytic action of a phenolic acid decarboxylase (PAD) to give a desired optionally substituted *p*-vinyl phenol **4**;
d) wherein the generated CO₂ is removed from the reaction system, in order to shift the chemical equilibrium of all three reaction steps towards the product sides.

2. The method according to claim 1, **characterized in that** the reaction is conducted at a pH of about 8 to 9.

3. The method according to claim 2, **characterized in that** the reaction is conducted at a pH of about 8.

4. The method according to any one of claims 1 to 3, **characterized in that**, in step b), a tyrosine ammonia-iyase (TAL) is used in the form of whole cells containing the recombinant enzyme.

5. The method according to any one of claims 1 to 5, **characterized in that**, in step c), a ferulic acid decarboxylase (FAD) is used as the catalyst.

6. The method according to claim 5, **characterized in that** the ferulic acid decarboxylase (FAD) is used in the form of whole cells containing the recombinant enzyme.

7. The method according to any one of claims 1 to 7, **characterized in that** the one-pot reaction is conducted in an aqueous buffer system in the presence of a water-immiscibie co-solvent.

8. The method according to claim 7, **characterized in that** diethyl ether is used as the co-solvent, preferably in an amount of 5% (v/v) based on the aqueous buffer system.

9. The method according to any one of the preceding claims, **characterized in that** the phenol **1** is substituted in the *ortho-* and/or *meta*-position(s) with one or more substituents R selected from halogens, C₁₋₆ alkyl, and C₁₋₆ alkoxy.

## Revendications

1. Procédé biocatalytique pour produire des para-vinylphénols, comprenant une synthèse monotope à trois étapes selon le schéma réactionnel ci-dessus: 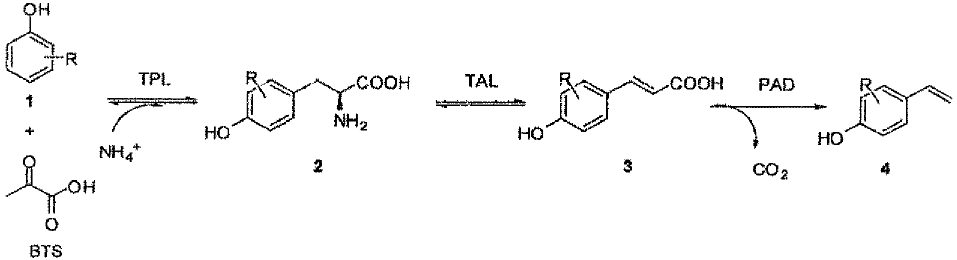 dans lequel
a) un phénol 1, éventuellement substitué, est lié à l'acide pyruvique (BTS) par action catalytique d'une tyrosine-phénol-lyase (TPL) et en présence de ions d'ammonium pour obtenir une tyrosine 2, éventuellement substituée,
b) ammoniac est éliminé de la tyrosine 2 par action catalytique d'une tyrosine-ammoniac-lyase (TAL) pour donner un acide paracoumarique 3, éventuellement substitué,
c) l'acide paracoumarique 3 est soumis à décarboxylation par action catalytique d'une décarboxylase de l'acide phénolique (PAD) pour donner un para-vinylphénol souhaité, éventuellement substitué,
d) le CO₂ généré est éliminé du système de réaction pour déplacer l'équilibre chimique des trois étapes de réaction vers les produits.

2. Procédé selon la revendication 1, **caractérisé en ce que** la réaction est effectuée à un pH d'environ 8 à 9.

3. Procédé selon la revendication 2, **caractérisé en ce que** la réaction est effectuée à un pH d'environ 8.

4. Procédé selon l'une quelconque des revendications 1 à 3, **caractérisé en ce que** dans l'étape b) la tyrosine-ammoniac-lyase (TAL) est employée sous forme de cellules entières contenant l'enzyme recombinante.

5. Procédé selon l'une quelconque des revendications 1 à 4, **caractérisé en ce que** dans l'étape c) est utilisé comme catalyseur une décarboxylase de l'acide férulique (FAD).

6. Procédé selon la revendication 5, **caractérisé en ce que** la décarboxylase de l'acide férulique (FAD) est employée sous forme de cellules entières contenant l'enzyme recombinante.

7. Procédé selon l'une quelconque des revendications 1 à 6, **caractérisé en ce que** la réaction monotope est effectuée dans un système tampon aqueux en présence d'un co-solvant non miscible à l'eau.

8. Procédé selon la revendication 7, **caractérisé en ce que** comme co-solvant est utilisé le diéthyléther, de préférence dans une quantité de 5 % en volume par rapport au système tampon aqueux.

9. Procédé selon l'une quelconque des revendications précédentes, **caractérisé en ce que** le phénol 1 est substitué en position ortho et/ou méta par un ou plusieurs substituants R qui sont choisis parmi des halogènes, un
